(19) Europäisches Patentamt / European Patent Office / Office européen des brevets

(11) **EP 4 384 802 B1**

(12) **EUROPÄISCHE PATENTSCHRIFT**

(45) Veröffentlichungstag und Bekanntmachung des Hinweises auf die Patenterteilung:
**27.08.2025   Patentblatt 2025/35**

(21) Anmeldenummer: **22741809.2**

(22) Anmeldetag: **22.07.2022**

(51) Internationale Patentklassifikation (IPC):
**G01N 21/27** *(2006.01)*   **G01N 21/31** *(2006.01)*
**G01N 21/82** *(2006.01)*   **G01N 33/86** *(2006.01)*
**G01N 33/49** *(2006.01)*

(52) Gemeinsame Patentklassifikation (CPC):
**G01N 21/82; G01N 21/272; G01N 21/31;**
**G01N 33/491; G01N 33/86;** G01N 21/314;
G01N 2021/825

(86) Internationale Anmeldenummer:
**PCT/EP2022/070619**

(87) Internationale Veröffentlichungsnummer:
**WO 2023/016778 (16.02.2023 Gazette 2023/07)**

(54) **VERFAHREN ZUR ERSTELLUNG EINER DATENBANK ZUR ERMITTLUNG EINES LICHTTRANSMISSIONSAGGREGOMETRIE-REFERENZWERTS UND VERFAHREN UND VORRICHTUNG ZUR DURCHFÜHRUNG EINER LICHTTRANSMISSIONSAGGREGOMETRIE-MESSUNG**

METHOD FOR CREATING A DATABASE FOR DETERMINING A LIGHT TRANSMISSION AGGREGOMETRY REFERENCE VALUE, AND METHOD AND DEVICE FOR CARRYING OUT A LIGHT TRANSMISSION AGGREGOMETRY MEASUREMENT

PROCÉDÉ DE CRÉATION D'UNE BASE DE DONNÉES POUR DÉTERMINER UNE VALEUR DE RÉFÉRENCE D'AGGRÉGOMÉTRIE À TRANSMISSION DE LUMIÈRE ET PROCÉDÉ ET DISPOSITIF DE MISE EN OEUVRE D'UNE MESURE D'AGGRÉGOMÉTRIE À TRANSMISSION DE LUMIÈRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **10.08.2021   EP 21190636**

(43) Veröffentlichungstag der Anmeldung:
**19.06.2024   Patentblatt 2024/25**

(73) Patentinhaber: **Kommanditgesellschaft Behnk Elektronik GmbH & Co.**
**22851 Norderstedt (DE)**

(72) Erfinder: **RADON, Christian**
 **22393 Hamburg (DE)**

(74) Vertreter: **Glawe, Delfs, Moll**
**Partnerschaft mbB**
**Hopfenmarkt 33**
**20457 Hamburg (DE)**

(56) Entgegenhaltungen:
**US-A1- 2017 248 576**

• **SAKAYORI TASUKU ET AL: "Evaluation of the Newly Developed Adenosine Diphosphate-Induced Platelet Aggregation Level System in Aggregometer on Automated Coagulation Analyzer", vol. 65, no. 12/2019, December 2019 (2019-12-01), DE, XP055871304, ISSN: 1433-6510, Retrieved from the Internet <URL:http://dx.doi.org/10.7754/Clin.Lab.2019.190353> DOI: 10.7754/Clin.Lab.2019.190353**

- **LING LI-QIN ET AL: "Evaluation of an automated light transmission aggregometry", vol. 28, no. 7, 2 February 2017 (2017-02-02), GB, pages 712 - 719, XP055871233, ISSN: 0953-7104, Retrieved from the Internet <URL:https://www.tandfonline.com/doi/pdf/10.1080/09537104.2016.1265923> DOI: 10.1080/09537104.2016.1265923**
- **LE BLANC JESSICA ET AL: "Advances in Platelet Function Testing-Light Transmission Aggregometry and Beyond", JOURNAL OF CLINICAL MEDICINE, vol. 9, no. 8, 13 August 2020 (2020-08-13), pages 2636, XP055871250, DOI: 10.3390/jcm9082636**
- **MUKAIDE KAE: "Overview of the Automated Coagulation Analyzer CS5100", SYSMEX JOURNAL INTERNATIONAL, 2013, XP055871951, Retrieved from the Internet <URL:https://www.sysmex.co.jp/en/products_solutions/library/journal/vol23_no1/vol23_1_02.pdf> [retrieved on 20211213]**

**Beschreibung**

Gegenstand der Erfindung ist ein Verfahren zur Erstellung einer Datenbank, die zur Ermittlung eines virtuellen Referenzwerts für einen PPP-Referenzwert einer Lichttransmissionsaggregometrie-Messung verwendet werden kann. Gegenstand der Erfindung ist weiterhin ein Verfahren zur Ermittlung eines virtuellen Referenzwerts für einen PPP-Referenzwert einer Lichttransmissionsaggregometrie-Messung am plättchenreichen Plasma einer zu untersuchenden Blutprobe unter Verwendung der erstellten Datenbank sowie die Durchführung einer Lichttransmissionsaggregometrie-Messung unter Verwendung des virtuellen Referenzwerts.

Zur Beurteilung der Funktionsfähigkeit von Thrombozyten (Blutplättchen) ist die Lichttransmissionsaggregometrie (LTA) eines der am häufigsten verwendeten Verfahren. Für die Durchführung einer LTA-Messung wird aus einer von einem Probanden bereitgestellten Blutprobe mittels Zentrifugation sogenanntes plättchenreiches Plasma (PRP) gewonnen. Aufgrund ihrer scheibenförmigen Gestalt mit einem Durchmesser zwischen 1,5 $\mu$m und 3 $\mu$m findet an den Thrombozyten Lichtstreuung statt, die das PRP als trübe Flüssigkeit erscheinen lässt. Nach der Zugabe eines Aktivators zu der PRP-Probe vernetzen sich die Thrombozyten und bilden Aggregate, wodurch die Lichtdurchlässigkeit der PRP-Probe (zumindest soweit diese von gesunden Probanden stammt) zunimmt und der Transmissionsgrad sich im Zeitverlauf einem Maximum annähert. Im Rahmen einer LTA-Messung wird unter standardisierten Bedingungen diese Zunahme des Transmissionsgrades (bzw. die Abnahme der Extinktion) im Zeitverlauf vermessen, indem ein Lichtstrahl auf die PRP-Probe gerichtet und die Intensität des aus der Probe heraustretenden Lichtstrahls gemessen wird. Das Dokument "SAKAYORI TASKUKU ET AL: "Evaluation of the Newly Developed Adenosine Diphosphate induced Platelet Aggregation Level System in Aggregometer on Automated Coagulation Analyzer", CLINICAL LABORATORY, Bd. 65, Nr. 12/2019, 1. Dezember 2019 (2019-12-01)" offenbart ein LTA-Verfahren, bei dem ein sogenanntes "ADP-induziertes Plättchen-Aggregations-Niveau" (ADP-induced platelet aggregation level (APAL)) zur Auswertung der LTA-Messung verwendet wird. In "LING LI-QIN ET AL: "Evaluation of an automated light transmission aggregometry", PLATELETS (LONDON), Bd. 28, Nr. 7, 2. Februar 2017 (2017-02-02), Seiten 712-719" wird eine Studie zur Leistungsfähigkeit eines Koagulations-Analysegeräts vorgestellt. Im Dokument "LE BLANC JESSICA ET AL: "Advances in Platelet Function Tenting-Light Transmission Aggregometry and Beyond", JOURNAL OF CLINICAL MEDICINE, Bd. 9, Nr. 8, 13. August 2020 (2020-08-13), Seite 2636" wird ein einen Überblick gegeben über neue Entwicklungen im Bereich der LTA-Messverfahren. US 2017/248576 A1 offenbart ein LTA-Analysegerät, welches drei Lichtquellen zur Aussendung von drei verschiedenen Lichtwellenlängen aufweist, wobei die verschiedenen Wellenlängen für unterschiedliche Messaufgaben verwendet werden. Das Dokument "Mukaide Kae: "Overview of the Automated Coagulation Analyzer CS5100', Sysmex Journal International, 2013" liefert eine Produktübersicht über das automatische Gerinnungsanalysegerät "CS-5100".

Um die Änderung der Lichttransmission interpretieren zu können, ist es im Stand der Technik erforderlich, zusätzlich zur PRP-Probe eine Probe plättchenarmen Plasmas (auf Englisch: "platelet-poor plasma", nachfolgend "PPP") desselben Probanden bereitzustellen. Von dieser PPP-Probe wird ebenfalls der Transmissionsgrad bestimmt und als Referenzwert für die am PRP vorgenommene LTA-Messung verwendet. Da das PPP nur eine geringe Thrombozytenkonzentration aufweist, ist es üblicherweise eine klare Flüssigkeit und weist einen maximalen Transmissionsgrad auf. Der am PRP gemessene Transmissionsgrad wird üblicherweise zu diesem maximalen Transmissionsgrad ins Verhältnis gesetzt. Erst diese Vorgehensweise ermöglicht es im Stand der Technik, die LTA-Messung sinnvoll zu interpretieren und insbesondere das maximale Ausmaß der Aggregation sowie die Geschwindigkeit der Aggregationsänderung beurteilen zu können.

Die erforderliche Entnahme einer zusätzlichen Blutmenge zur Gewinnung der PPP-Probe kann für den Probanden belastend sein. Dies gilt insbesondere für Neugeborene oder Säuglinge, die von Natur aus eine geringe Blutmenge aufweisen, oder auch für Patienten, denen aufgrund von Vorerkrankungen nur eingeschränkt Blut abgenommen werden kann. Darüber hinaus sind für die Bereitstellung der PPP-Probe zusätzlicher Arbeitsaufwand sowie zusätzliche Verbrauchsmaterialien erforderlich.

Vor diesem Hintergrund ist es die Aufgabe der vorliegenden Erfindung ein Verfahren zur Erstellung einer Datenbank zur Ermittlung eines virtuellen Referenzwerts für eine LTA-Messung, ein Verfahren zur Ermittlung eines virtuellen Referenzwerts für die Durchführung einer LTA-Messung unter Verwendung der erstellten Datenbank sowie ein Verfahren zur Durchführung einer LTA-Messung bereitzustellen, welche zuverlässige Ergebnisse mit geringerem Aufwand liefern. Gelöst wird diese Aufgabe mit Hilfe der Merkmale der unabhängigen Ansprüche. Vorteilhafte Ausführungsformen sind in den Unteransprüchen angegeben.

Das erfindungsgemäße Verfahren zur Erstellung einer Datenbank zur Ermittlung eines virtuellen Referenzwerts für einen PPP-Referenzwert einer LTA-Messung ist im Anspruch 1 definiert und umfasst die nachfolgenden Schritte:

a. Bereitstellen von plättchenreichem Plasma (PRP) einer Referenzblutprobe;

b. Durchführen einer Lichttransmissionsmessung mit einer ersten Lichtwellenlänge und einer zweiten von der ersten verschiedenen Lichtwellenlänge am PRP der Referenzblutprobe;

c. Bereitstellen von plättchenarmem Plasma (PPP) der Referenzblutprobe;

d. Durchführen einer Lichttransmissionsmessung am PPP zur Ermittlung eines PPP-Referenzwerts;

e. Zuordnen des Messergebnisses des Schrittes d zu den Messergebnissen des Schrittes b in einer Datenbank;

f. Wiederholen der Schritte a bis e für eine Mehrzahl von Referenzblutproben.

Zunächst werden einige im Rahmen der vorliegenden Beschreibung verwendete Begriffe erläutert. Das erfindungsgemäße Verfahren erfordert die Bereitstellung von plättchenreichem und plättchenarmem Plasma einer Blutprobe. Die Bereitstellung kann auf grundsätzlich bekannte Weise, beispielsweise wie in dem Dokument "Leitlinie-Thrombozytopathien, Version 2.1 (AWMF-Register Nr. 086-003, update 2/2018)" beschrieben, erfolgen. Dabei wird der Blutprobe insbesondere eine definierte Menge einer geeigneten Antikoagulanz (beispielsweise Natriumcitrat) beigegeben, um eine Blutgerinnung zu verhindern.

Bei der Durchführung einer Lichttransmissionsmessung am PRP bzw. am PPP der Blutprobe wird vorzugsweise ein Lichtstrahl einer vorgegebenen Intensität auf ein üblicherweise in einem durchsichtigen Behälter befindliches Volumen des PRP bzw. des PPP gerichtet, wobei die Intensität des Lichtstrahls nach dem Durchtritt durch das Volumen gemessen wird. Aus dem Verhältnis der anfänglich vorgegebenen Intensität und der Intensität nach dem Durchtritt ergibt sich ein Transmissionsgrad, der das Ergebnis der Lichttransmissionsmessung darstellen kann. Im Falle einer Lichttransmissionsmessung am PPP kann der Transmissionsgrad den PPP-Referenzwert bilden.

Wenn Lichttransmissionsmessungen mit einer ersten Wellenlänge und mit einer zweiten Wellenlänge durchgeführt werden, können diese Messungen zeitlich nacheinander oder auch gleichzeitig an unterschiedlichen Teilvolumina des untersuchten Volumens durchgeführt werden.

Zwei Wellenlängen werden im Rahmen der vorliegenden Beschreibung als verschieden angesehen, wenn sie sich um mindestens 10 nm unterscheiden. Erfindungsgemäß unterscheidet sich die erste Wellenlänge von der zweiten Wellenlänge um mindestens 50 nm. In einer Ausführungsform kann sich die erste Wellenlänge von der zweiten Wellenlänge um mindestens 100 nm, vorzugsweise um mindestens 200 nm unterscheiden. Die erste Wellenlänge liegt erfindungsgemäß in einem Bereich zwischen 300 nm und 500 nm und liegt vorzugsweise zwischen 345 nm und 465 nm, weiter vorzugsweise zwischen 385 nm und 425 nm. Die zweite Wellenlänge liegt erfindungsgemäß in einem Bereich zwischen 500 nm und 800 nm und liegt vorzugsweise zwischen 550 nm und 700 nm, weiter vorzugsweise wischen 600 nm und 640 nm.

Die am PRP vorgenommenen Lichttransmissionsmessungen können im Rahmen der Erfindung mit monochromatischem Licht durchgeführt werden, das zumindest den oben genannten Wellenlängenabstand aufweist bzw. innerhalb den oben genannten Bereiche liegt. Möglich ist es im Rahmen der Erfindung auch, dass durch eine Überlagerung mehrerer Lichtwellenlängen gebildete Lichtbündel für die Lichttransmissionsmessung verwendet werden. Die Wellenlänge des Lichtbündels ist in diesem Fall durch einen (vorzugsweise nach der Intensität gewichteten) Mittelwert der im Lichtbündel enthaltenen Wellenlängen gegeben. Der Wellenlängenabstand zweier Lichtbündel ist insoweit durch den Abstand der Mittelwerte der Lichtbündel vorgegeben. Wenn im Rahmen der vorliegenden Beschreibung vom "Licht einer Wellenlänge" die Rede ist, kann damit auch ein entsprechendes Lichtbündel gemeint sein, dessen Wellenlängenmittelwert bei dieser Wellenlänge liegt.

Im Rahmen der Erfindung wird am PRP einer Referenzblutprobe eine Lichttransmissionsmessung mit zumindest zwei verschiedenen Wellenlängen durchgeführt, aus der insbesondere ein Transmissionsgrad ermittelt werden kann. Zusätzlich wird am PPP der Referenzblutprobe auf aus dem Stand der Technik grundsätzlich bekannte Weise ein Referenzwert der Lichttransmission ermittelt (nachfolgend auch PPP-Referenzwert genannt). Im Rahmen der Erfindung wurde erkannt, dass zwischen den am PRP mit den zumindest zwei verschiedenen Wellenlängen gewonnenen Messwerten und dem PPP-Referenzwert ein statistisch signifikanter Zusammenhang besteht, welcher zum Vorschein tritt, wenn man die in der Datenbank abgelegten Werte für eine Mehrzahl von Referenzblutproben auswertet. Dieser statistische Zusammenhang ermöglicht es, nach der Erstellung der Datenbank diese zu verwenden, um für eine weitere zu untersuchende Blutprobe eines Patienten einen virtuellen Referenzwert zu ermitteln, welcher einen zuverlässigen Schätzwert des PPP-Referenzwerts darstellt, ohne dass von demselben Patienten zusätzlich eine PPP-Probe gewonnen und vermessen werden muss. Beispielsweise kann aus der Datenbank ein mathematischer Zusammenhang zwischen den am PRP einer zu untersuchenden Blutprobe erhaltenen Messwerten und dem virtuellen Referenzwert ermittelt werden. Dies wird nachfolgend in Verbindung mit dem Verfahren zur Ermittlung eines virtuellen Referenzwerts sowie dem Verfahren zur Durchführung einer LTA-Messung noch im Detail erläutert. Alternativ kann aus der Datenbank auch mit anderen Mitteln, insbesondere mit Hilfe von grundsätzlich bekannten statistischen Verfahren (ggf. unter Zuhilfenahme von künstlicher Intelligenz) ein virtueller Referenzwert für einen PPP-Referenzwert ermittelt werden. Solche Verfahren sind dem Fachmann grundsätzlich bekannt. Der Kern der Erfindung liegt in der Erkenntnis, dass sich durch die erfindungsgemäßen Verfahrensschritte eine Datenbank erstellen lässt, in der ausreichend Informationen zur Ermittlung des virtuellen Referenzwerts vorhanden sind. Aufgrund der erfindungsgemäßen Datenbank kann daher auf eine zusätzliche Blutabnahme zur Gewinnung einer PPP-Probe verzichtet und der dadurch verursachte Aufwand vermieden werden.

Im Rahmen der Erfindung wurde erkannt, dass für eine konkrete Blutprobe sowohl der PPP-Referenzwert als auch die mit verschiedenen Wellenlängen am PRP gewonnenen Messwerte abhängig sind vom physiologischen Zustand der Blutprobe, also insbesondere von der Art und Konzentration von in der Blutprobe enthaltenen Inhaltsstoffen, welche den Transmissionsgrad sowohl der PPP-Probe (und damit den PPP-Referenwert) als auch der PRP-Probe beeinflussen. Solche Inhaltsstoffe können beispielsweise Hämoglobin, Ceruloplasmin, Bilirubin, Lipoproteine, oder auch sonstige durch Medikamente zugeführte Inhaltsstoffe sein. Die Erfinder gehen davon aus, dass es bestimmte Inhaltsstoffe gibt, die den Transmissionsgrad in Abhängigkeit der Lichtwellenlänge beeinflussen. Durch die Verwendung von zumindest zwei verschiedenen Lichtwellenlängen ergibt sich somit innerhalb der Datenbank eine Korrelation zwischen den am PRP ermittelten Messwerten und dem PPP-Referenzwert, da sowohl die PRP-Messwerte als auch der PPP-Referenzwert auf vorbestimmte Weise von der Art und/oder Konzentration der in der Blutprobe enthaltenen Inhaltsstoffe beeinflusst werden. Nach Erstellung der Datenbank kann daher bei der Untersuchung von weiteren Blutproben allein durch Vermessung des PRP mit den verschiedenen Wellenlängen und ggf. unter Verwendung der Datenbank (bzw. eines daraus erhaltenen mathematischen Zusammenhangs) ein Rückschluss auf den PPP-Referenzwert gezogen werden, ohne dass eine zusätzliche PPP-Probe gewonnen werden muss.

In einer bevorzugten Ausführungsform umfasst das Verfahren zur Erstellung der Datenbank die weiteren Schritte:

g. Zugeben einer vorgegebenen Menge eines Aktivators zum PRP der Referenzblutprobe nach dem Durchführen der Messung gemäß Schritt b;

h. Wiederholen der Messung gemäß Schritt b nach dem Zugeben des Aktivators und vor dem Einsetzen einer durch den Aktivator ausgelösten Aggregation;

i. Zuordnen des Messergebnisses des Schrittes h zu dem Messergebnis des Schrittes d in der Datenbank;

j. Wiederholen der Schritte g bis i für die Mehrzahl von Referenzblutproben.

Der Begriff "Aktivator" bezeichnet dabei ein Reagens oder eine Mehrzahl von Reagenzien, das oder die dazu eingerichtet ist/sind, nach der Zugabe zum PRP eine Aggregation der Thrombozyten auszulösen. Der Aktivator kann eines oder mehrere Reagenzien umfassen, die ausgewählt sind aus der Gruppe umfassend Ristocetin, Arachidonsäure, Adenosintriphosphat (ADP), Epinephrin (Adrenalin), Kollagen, Thrombin-Rezeptor-aktivierende Peptide (TRAPs). Nach dem Zugeben des Aktivators setzt die Aggregation mit einer Zeitverzögerung ein. Die Thrombozyten verbleiben über einen gewissen Zeitraum im aktivierten Zustand und es findet noch keine Zunahme der Lichttransmission statt, die durch eine Vernetzung der Thrombozyten ausgelöst wird. Vorzugsweise wird während dieses Zeitraums, also bevor die eigentliche Aggregation stattfindet, die wiederholte Messung des Schritts h durchgeführt. Die Lichttransmissionsmessung nach Zugabe des Aktivators gemäß Schritt h wird vorzugsweise in einem Zeitraum zwischen 0 und 10s, vorzugsweise zwischen 0 und 5s nach der Zugabe des Aktivators durchgeführt, wobei vorzugsweise zur Erfassung eines Transmissionswertes ein zeitlicher Mittelwert über einen Zeitraum zwischen 1s und 6s, vorzugsweise zwischen 3s und 5s gebildet wird. Die Lichttransmissionsmessung vor der Zugabe des Aktivators findet vorzugsweise unmittelbar vor der Zugabe des Aktivators statt, beispielsweise in einem Zeitraum zwischen 0s und 10s vor der Zugabe des Aktivators. Das Mischungsverhältnis zwischen einem PRP-Volumen und dem Volumen des Aktivators, das dem PRP-Volumen gemäß Schritt g zugegeben wird, liegt üblicherweise bei 9:1, kann aber beispielsweise auch zwischen 20:1 und 2:1, vorzugsweise zwischen 15:1 und 4:1 weiter vorzugsweise zwischen 7:1 und 11:1 liegen.

Bei der vorstehend beschriebenen Ausführungsform wird die Lichttransmissionsmessung mit zwei Lichtwellenlängen sowohl vor als auch unmittelbar nach der Zugabe einer vorgegebenen Menge des Aktivators durchgeführt. Es hat sich gezeigt, dass durch die zusätzlich gewonnenen Messwerte noch genauere und aussagekräftigere Rückschlüsse auf einen PPP-Referenzwert gezogen werden können. Insbesondere wurde erkannt, dass die Zugabe einer vorgegebenen Menge des Aktivators zum PRP eine initiale und von der individuellen PRP-Probe abhängige Veränderung der Lichttransmission hervorruft, bei der es sich um eine Abnahme oder auch eine Zunahme der Lichttransmission handeln kann. Diese initiale Veränderung weist ebenfalls eine Abhängigkeit vom physiologischen Zustand der Blutprobe, und insbesondere von deren Inhaltsstoffen auf. In der beschriebenen Ausführungsform werden der Datenbank somit zusätzliche Informationen hinzugefügt, welche einen verbesserten statistischen Rückschluss auf einen PPP-Referenzwert ermöglichen.

In einer Ausführungsform der Erfindung ist vorgesehen, dass im Verfahrensschritt g bei einer ersten Referenzblutprobe ein erster Aktivator und bei einer zweiten Referenzblutprobe ein zweiter vom ersten verschiedener Aktivator verwendet wird. Dadurch wird es möglich, innerhalb der Datenbank zwischen Messdaten, die mittels verschiedener Aktivatoren erhalten wurden, zu unterscheiden. Wenn später unter Verwendung eines bestimmten Aktivators ein virtueller Referenzwert einer zu untersuchenden Blutprobe ermittelt wird, kann dann innerhalb der Datenbank auf Messergebnisse, die an Referenzblutproben unter Verwendung desselben Aktivators erfasst wurden (bzw. auf daraus ermittelte mathematische Zusammenhänge) zurückgegriffen werden. Wenn zu erwarten ist, dass die verschiedenen Aktivatoren keine unterschiedlichen Messergebnisse hervorrufen, können die anhand verschiedener Aktivatoren erhaltenen Messergebnisse auch in der Datenbank zusammengefasst werden bzw. gemeinsam zur Ermittlung eines mathematischen Zusammenhangs verwendet werden.

Die Lichttransmissionsmessungen des Schrittes b und/oder des Schrittes h können mit zumindest drei voneinander verschiedenen Lichtwellenlängen durchgeführt werden. Insbesondere kann das Licht der ersten Wellenlänge eine Wellenlänge im Bereich zwischen 380 nm bis 420 nm, vorzugsweise zwischen 400 nm und 410 nm aufweisen. Licht der zweiten Wellenlänge kann eine Wellenlänge im Bereich zwischen 500 nm bis 550 nm, vorzugsweise zwischen 520 nm und 530 nm aufweisen. Licht einer dritten Wellenlänge kann eine Wellenlänge im Bereich zwischen 620 nm bis 700 nm, vorzugsweise zwischen 620 nm und 630 nm aufweisen. Es hat sich gezeigt, dass durch die Verwendung von drei Wellenlängen die Genauigkeit, mit der ein Rückschluss auf einen PPP-Referenzwert gezogen werden kann, weiter erhöht wird. Die drei Wellenlängen werden vorzugsweise sowohl für die Messung vor der Zugabe als auch für die Messung nach der Zugabe des Aktivators verwendet.

In einer vorteilhaften Ausführungsform weist die Mehrzahl von Referenzblutproben eine erste Referenzblutprobe und ein zweite Referenzblutprobe auf, wobei die erste Referenzblutprobe zumindest einen ersten Inhaltsstoff aufweist, der in der zweiten Referenzblutprobe nicht oder in geringerer Konzentration enthalten ist. Beispielsweise kann die Konzentration des Inhaltsstoffs in der ersten Referenzblutprobe um einen Faktor von mehr als 1,5, vorzugsweise mehr als 3, weiter vorzugsweise mehr als 5 größer sein als die Konzentration des Inhaltsstoffs in der zweiten Referenzblutprobe. Möglich sind auch Konzentrationsunterschiede um beispielsweise einen Faktor von 20. Der erste Inhaltsstoff kann vorzugsweise ausgewählt sein aus der Gruppe bestehend aus Hämoglobin, Ceruloplasmin, Lipoprotein, Triglyceride, Bilirubin. Es kann sich beim ersten Inhaltsstoff auch um Farbstoffe oder Bestandteile handeln, die aus Medikamenten oder der Nahrung stammen, oder um deren Abbauprodukte. Wenn sich die Referenzblutproben hinsichtlich ihrer Inhaltsstoffe unterscheiden, deckt die Datenbank eine breitere Basis an unterschiedlichen physiologischen Zuständen der Blutproben ab. Erfindungsgemäß werden zur Erstellung der Datenbank eine Mehrzahl von Referenzblutproben (beispielsweise mehr als 5, vorzugsweise mehr als 10, weiter vorzugsweise mehr als 20) verwendet, die sich hinsichtlich der Art und/oder Konzentration von zumindest einem ihrer Inhaltsstoffe, beispielsweise durch die oben beschriebenen Konzentrationsunterschiede, jeweils paarweise voneinander unterscheiden. Möglich ist auch, dass die Datenbank unter Verwendung von Referenzblutproben erstellt wird, die sich jeweils paarweise durch mehr als einen, vorzugsweise mehr als 2, weiter vorzugsweise mehr als 5 Inhaltsstoffen, voneinander unterscheiden, wobei der Unterschied durch die oben genannte Konzentrationsdifferenz gegeben sein kann. Die Genauigkeit, mit der aus der Datenbank statistische Rückschlüsse gezogen werden können, wird dadurch weiter verbessert.

Es kann vorgesehen sein, dass das PRP-Volumen einer Referenzblutprobe in eine Mehrzahl von Teilvolumina aufgeteilt wird, wobei das erfindungsgemäße Verfahren an jedem dieser Teilvolumina durchgeführt wird, wobei anschließend über die Anzahl der Teilvolumina jeweils ein Mittelwert der ermittelten Messwerte gebildet und in die Datenbank aufgenommen wird. Durch eine solche Mittelwertbildung kann die statistische Genauigkeit weiter erhöht werden.

In einer Ausführungsform wird der erste Inhaltsstoff der ersten Referenzblutprobe manuell hinzugefügt. Möglich ist auch, verschiedenen Referenzblutproben den Inhaltsstoff in verschiedenen vorgegebenen Konzentrationen zuzuführen. Dies hat den Vorteil, dass der Einfluss eines Inhaltsstoffs auf die im Rahmen der Erfindung ermittelten Messwerte systematisch erfasst und in die Datenbank aufgenommen werden kann. Insbesondere können Messreihen mit einer Mehrzahl von Referenzblutproben vorgenommen werden, bei denen der Inhaltsstoff verschiedenen Referenzblutproben in einer Mehrzahl von unterschiedlichen Konzentrationen zugegeben wird und/oder bei denen verschiedenen Referenzblutproben eine Mehrzahl von verschiedenen Inhaltsstoffen ggf. in unterschiedlichen Konzentrationen zugegeben wird.

Möglich ist grundsätzlich auch, dass zumindest ein Teil der für die Durchführung des erfindungsgemäßen Verfahrens verwendeten Referenzblutproben von Probanden gewonnen sind, bei denen, beispielsweise aufgrund von Vorerkrankungen oder der Einnahme von Medikamenten oder sonstigen Einflüssen, damit zu rechnen ist, dass die Referenzblutproben sich in der Art und/oder der Konzentration der darin enthaltenen Inhaltsstoffe voneinander unterscheiden.

Gegenstand der Erfindung ist weiterhin ein Verfahren gemäß Anspruch 9 zur Ermittlung eines virtuellen Referenzwerts für die Durchführung einer LTA-Messung am PRP einer zu untersuchenden Blutprobe unter Verwendung einer erfindungsgemäßen Datenbank. Das Verfahren umfasst die nachfolgenden Schritte:

a. Bereitstellen von PRP der zu untersuchenden Blutprobe;

b. Durchführen einer Lichttransmissionsmessung mit einer ersten Lichtwellenlänge und einer zweiten von der ersten verschiedenen Lichtwellenlänge am PRP der zu untersuchenden Blutprobe;

c. Verwenden der durch den Schritt b erhaltenen Messergebnisse sowie der erfindungsgemäßen Datenbank zur Ermittlung des virtuellen Referenzwerts der zu untersuchenden Blutprobe.

Nachdem eine erfindungsgemäße Datenbank erstellt wurde, kann mit dem vorstehend beschriebenen Verfahren für eine weitere zu untersuchende Blutprobe ein sogenannter virtueller Referenzwert ermittelt werden. Der virtuelle Referenzwert stellt vorliegend einen anhand der Datenbank und den am PRP vorgenommenen Messungen ermittelten Schätzwert des PPP-Referenzwerts dar. Mit Hilfe des virtuellen Referenzwerts kann eine anschließend durchgeführte LTA-Messung sinnvoll interpretiert werden, ohne dass eine PPP-Probe desselben Patienten gewonnen werden muss.

Vorzugsweise umfasst das Verfahren zur Ermittlung eines virtuellen Referenzwerts die weiteren Schritte:

d. Zugeben einer vorgegebenen Menge eines Aktivators zum PRP der zu untersuchenden Blutprobe nach dem Durchführen der Messung gemäß Schritt b;

e. Wiederholen der Messung gemäß Schritt b nach dem Zugeben des Aktivators zum PRP der zu untersuchenden Blutprobe und vor dem Einsetzen einer durch den Aktivator ausgelösten Aggregation;

f. Einbeziehen der durch den Schritt e erhaltenen Messergebnisse in die Ermittlung des Schrittes c.

Das Bereitstellen des PRP gemäß Schritt a sowie die Durchführung der Lichttransmissionsmessungen gemäß den Schritten b und/oder e kann auf dieselbe Weise erfolgen wie oben in Verbindung mit dem Verfahren zur Erstellung der Datenbank beschrieben. Insbesondere kann vorgesehen sein, dass für die Messungen (zumindest im Wesentlichen) dieselben Lichtwellenlängen oder Lichtwellenlängenbereiche verwendet werden und/oder (zumindest im Wesentlichen) dasselbe Verhältnis zwischen dem PRP-Volumen und dem Volumen des zugegebenen Aktivators verwendet wird. Das Verfahren zur Ermittlung eines virtuellen Referenzwerts kann insoweit durch die oben bereits in Verbindung mit dem Verfahren zur Erstellung einer Datenbank beschriebenen Merkmale fortgebildet werden. Es kann dadurch sichergestellt werden, dass die Messergebnisse unter denselben oder zumindest möglichst ähnlichen Bedingungen wie bei der Erstellung der Datenbank erfasst werden. Dadurch kann der Rückschluss, der anhand der an der zu untersuchenden Blutprobe erfassten Messergebnisse gezogen wird, mit hoher Genauigkeit erfolgen.

Die Ermittlung des virtuellen Referenzwerts kann im einfachsten Fall durch einen Vergleich zwischen den Messergebnissen der zu untersuchenden Blutprobe (erhalten durch die Schritte b und ggf. e und den in der Datenbank enthaltenen Messergebnissen erfolgen. Falls in der Datenbank Messergebnisse einer Referenzblutprobe enthalten ist, die identisch oder im Wesentlichen identisch sind zu den Messergebnissen der zu untersuchenden Blutprobe, so kann der in der Datenbank zu dieser Referenzblutprobe vorhandene PPP-Referenzwert als virtueller Referenzwert verwendet werden.

In vielen Fällen wird sich jedoch in der Datenbank kein Datensatz einer Referenzblutprobe finden lassen, der mit dem Datensatz der zu untersuchenden Blutprobe in ausreichendem Maße übereinstimmt. Zudem kann ein einzelner in der Datenbank enthaltener Datensatz grundsätzlich auch mit einem Messfehler behaftet sein, so dass der Rückgriff auf einen einzelnen Datensatz zur Bestimmung des virtuellen Referenzwerts fehleranfällig sein kann. Vorzugsweise ist daher vorgesehen, dass ein auf der Datenbank beruhender mathematischer Zusammenhang aufgestellt wird, der als Eingangsgrößen die an der zu untersuchenden Blutprobe gewonnenen Messergebnisse und als Ausgangsgröße den virtuellen Referenzwert der zu untersuchenden Blutprobe aufweist. Es hat sich insbesondere gezeigt, dass der virtuelle Referenzwert *virtPPP* (im Rahmen der Beschreibung teilweise auch als virtREF bezeichnet) als mathematische Funktion der Messwerte angegeben werden kann:

$$virtPPP = virtPPP(xd1, xd2, xd3, xd4),$$

wobei

*xd*1:    der am PRP der zu untersuchenden Blutprobe gemessene Transmissionsgrad bei der Wellenlänge $\lambda 1$ vor Zugabe des Aktivators,

*xd*2:    der am PRP der zu untersuchenden Blutprobe gemessene Transmissionsgrad bei der Wellenlänge $\lambda 2$ vor

Zugabe des Aktivators,

*xd*3:    der am PRP der zu untersuchenden Blutprobe gemessene Transmissionsgrad bei der Wellenlänge $\lambda$1 nach Zugabe des Aktivators,

*xd*4:    der am PRP der zu untersuchenden Blutprobe gemessene Transmissionsgrad bei der Wellenlänge $\lambda$2 nach Zugabe des Aktivators,

$\lambda$1:    die erste Wellenlänge, und

$\lambda$2:    die zweite Wellenlänge ist.

Der mathematische Zusammenhang bzw. die Funktion **virtPPP(*xd*1,*xd*2,*xd*3,*xd*4)** kann mittels grundsätzlich aus dem Stand der Technik bekannter statistischer Anpassungsverfahren aus der Datenbank gewonnen werden.

In einer vorteilhaften Ausführungsform werden im Rahmen des mathematischen Zusammenhangs die Verhältnisse **V1 = *xd*1/*xd*2, V2 = *xd*3/*xd*4,** und **V3 = V2/V1** ermittelt und für die Berechnung des virtuellen Referenzwerts verwendet.

Sofern mehr als zwei Lichtwellenlängen für die Erstellung der Datenbank und die Vermessung der zu untersuchenden Blutprobe verwendet wurden, kann die Funktion entsprechend mittels im Stand der Technik grundsätzlich bekannter statistischer Anpassungsverfahren erweitert werden.

Gegenstand der Erfindung ist weiterhin ein Verfahren gemäß Anspruch 12 zur Durchführung einer LTA-Messung an einer zu untersuchenden Blutprobe, umfassend die nachfolgenden Schritte:

a. Durchführen des erfindungsgemäßen Verfahrens zur Ermittlung eines virtuellen Referenzwerts der zu untersuchenden Blutprobe,

b. Durchführen einer LTA-Messung an der zu untersuchenden Blutprobe unter Verwendung des virtuellen Referenzwerts.

Insbesondere kann sich die Lichttransmissionsaggregometriemessung im Zeitverlauf unmittelbar an das Verfahren zur Ermittlung des virtuellen Referenzwerts anschließen. Wenn bei der Ermittlung des Referenzwerts bereits ein Aktivator beigegeben wurde, kann die dadurch verursachte Aggregation im Rahmen der sich anschließenden LTA-Messung erfasst werden. Der Aktivator ist vorzugsweise derart ausgebildet, dass er sich für eine LTA-Messung eignet. Die sich an das Verfahren zur Ermittlung des virtuellen Referenzwerts anschließende LTA-Messung kann grundsätzlich mit einer einzelnen Wellenlänge oder auch mit mehreren Wellenlängen erfolgen, die abwechselnd durch ein Volumen des PRP oder gleichzeitig durch verschiedene Teilvolumina des PRP geleitet werden.

Gegenstand der vorliegenden Erfindung ist weiterhin eine Vorrichtung gemäß Anspruch 13 zur Ermittlung eines virtuellen Referenzwerts für die Durchführung einer LTA-Messung am PRP einer zu untersuchenden Blutprobe, umfassend ein Beleuchtungsmodul zum wahlweisen Aussenden von Licht einer ersten sowie einer zweiten von der ersten verschiedenen Lichtwellenlänge, eine Probenaufnahme zum Einsetzen einer PRP-Probe derart, dass das Licht des Beleuchtungsmoduls durch das PRP hindurchtritt, einen Lichtsensor, der zur Erfassung des durch das PRP hindurchgetretenen Lichts ausgebildet ist, sowie ein Steuermodul, welches dazu ausgebildet ist, die Vorrichtung derart anzusteuern, dass die folgenden Schritte ausgeführt werden:

a. Durchführen einer Lichttransmissionsmessung mit einer ersten Lichtwellenlänge und einer zweiten von der ersten verschiedenen Lichtwellenlänge am PRP der zu untersuchenden Blutprobe;

b. Verwenden der durch den Schritt a erhaltenen Messergebnisse sowie einer erfindungsgemäßen Datenbank zur Ermittlung eines virtuellen Referenzwerts der zu untersuchenden Blutprobe.

In einer bevorzugten Ausführungsform umfasst die Vorrichtung einen Applikator zum automatischen Zugeben einer vorgegebenen Menge eines Aktivators zum PRP, wobei das Steuermodul dazu ausgebildet ist, die Vorrichtung derart anzusteuern, dass die folgenden Schritte ausgeführt werden:

c. Zugeben einer vorgegebenen Menge eines Aktivators zum PRP der zu untersuchenden Blutprobe nach dem Durchführen der Messung gemäß Schritt a;

d. Wiederholen der Messung gemäß Schritt a nach dem Zugeben des Aktivators zum PRP der zu untersuchenden Blutprobe und vor dem Einsetzen einer durch den Aktivator ausgelösten Aggregation;

e. Einbeziehen der durch den Schritt d erhaltenen Messergebnisse in die Ermittlung des Schrittes b.

Die Vorrichtung kann insbesondere ein Rechenmodul aufweisen, das zum Zugriff auf einen auf einer erfindungsgemäßen Datenbank beruhenden mathematischen Zusammenhangs und zur Ermittlung des virtuellen Referenzwerts auf Basis des mathematischen Zusammenhangs und der in Schritt a oder in den Schritten a und d erhaltenen Messwerte ausgebildet ist. Der mathematische Zusammenhang kann insbesondere in dem Rechenmodul abgelegt sein.

Die erfindungsgemäße Vorrichtung kann durch weitere Merkmale fortgebildet werden, die in Verbindung mit dem Verfahren zur Erstellung einer Datenbank, dem Verfahren zur Ermittlung eines virtuellen Referenzwerts und/oder dem Verfahren zur Durchführung einer Lichttransmissionsaggregometrie-Messung beschrieben wurden.

Nachfolgend werden bevorzugte Ausführungsbeispiele der Erfindung unter Bezugnahme auf die beigefügten Zeichnungen im Detail erläutert.

Es zeigen:

Figur 1: beispielhafte Lichttransmissionsmessungen, die am PRP von drei Referenzblutproben mit einer ersten Wellenlänge durchgeführt wurden;

Figur 2: die Lichttransmissionsmessungen der Figur 1, wobei die Messkurven zum Zeitpunkt t=-8s zum Nullpunkt verschoben wurden;

Figur 3: das Verhältnis zwischen den in Figur 2 gezeigten Transmissionsgraden zum jeweiligen PPP-Referenzwert der jeweiligen Referenzblutprobe im Zeitverlauf;

Figur 4: eine beispielhafte LTA-Messung, die am PRP einer zu untersuchenden Blutprobe durchgeführt wurde;

Figur 5: das Verhältnis zwischen dem in Figur 4 gezeigten Transmissionsgrad zum virtuellen Referenzwert sowie zum gemessenen PPP-Referenzwert der zu untersuchenden Blutprobe im Zeitverlauf;

Figur 6: einen Vergleich zwischen gemessenen PPP-Referenzwerten und mit dem erfindungsgemäßen Verfahren ermittelten virtuellen Referenzwerten für eine Vielzahl von zu untersuchenden Blutproben;

Figur 7: eine statistische Auswertung der in Figur 6 gezeigten Ergebnisse;

Figur 8: eine schematische Ansicht einer erfindungsgemäßen Vorrichtung zur Ermittlung eines virtuellen Referenzwerts.

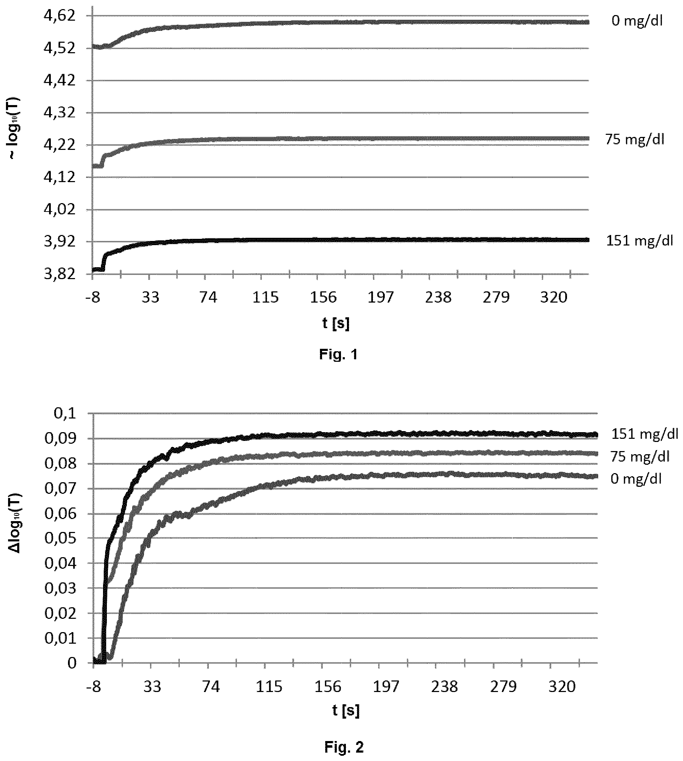

Figur 1 zeigt drei beispielhafte Lichttransmissionsmessungen im Zeitverlauf, die am PRP von drei verschiedenen Referenzblutproben durchgeführt wurden. Aufgetragen ist die Veränderung des Messwerts eines Intensitätsmessgerätes in Abhängigkeit von der Zeit. Der Messwert ist proportional zum dekadischen Logarithmus des Transmissionsgrades (der Transmissionsgrad wird nachfolgend mit dem Buchstaben T bezeichnet). Eine Zunahme des Messwertes entspricht also einer Zunahme des Transmissionsgrades. Die Messungen umfassen den Zeitraum t=-8s bis t=6 min, wobei zum Zeitpunkt t=0s der Aktivator Adenosindiphosphat (ADP) in einem Volumenverhältnis von 1:9 der jeweiligen PRP-Probe beigegeben wurde (ein Teil ADP, 9 Teile PRP). Die Messungen wurden mit einer Wellenlänge von $\lambda 1 = 625$ nm durchgeführt. Die Referenzblutproben 1 bis 3 unterscheiden sich voneinander lediglich in der Art eines der jeweiligen Probe manuell hinzugefügten Inhaltsstoffs. Während der Referenzblutprobe 1 kein Inhaltsstoff manuell zugegeben wurde, wurden den Referenzblutproben 2 und 3 Intralipide in einer Konzentration von 75 mg/dl bzw. 151 mg/dl zugegeben. In Figur 1 ist zu erkennen, dass die Zugabe der Intralipide zu einem geringeren Absolutwert des Transmissionsgrades führt.

Figur 2 zeigt die Messungen der Figur 1, wobei die Kurven zum Nullpunkt verschoben wurden, um die Veränderungen des Transmissionsgrades deutlicher zu machen. Es ist erkennbar, dass sich der Transmissionsgrad der Referenzprobe 1 kurz nach der Zugabe des Aktivators (zwischen t=0 und t=10s) kaum verändert oder sogar abnimmt. Während dieses Zeitraums setzt der Aktivierungsprozess der Thrombozyten ein, ohne dass bereits eine Aggregation stattfindet. Nach Ablauf des Zeitraums beginnt bei etwa t=10s die Vernetzung der Thrombozyten und es bilden sich Aggregate im PRP, wodurch der Transmissionsgrad im Zeitverlauf zunimmt. Nach etwa t=150s ist ein maximaler Transmissionsgrad erreicht, der sich danach kaum noch verändert.

Bei den Referenzproben 2 und 3 ist hingegen beim Zeitpunkt t=0 eine sprunghafte Veränderung um $\Delta$ **($\log_{10}$ $T$) = 0,03** bzw. um $\Delta$ **($\log_{10}$ $T$) = 0,05** zu erkennen. Auch bei diesen Referenzblutproben beginnt anschließend (ab etwa t=10s) die Aggregation und der Transmissionsgrad nimmt weiter zu.

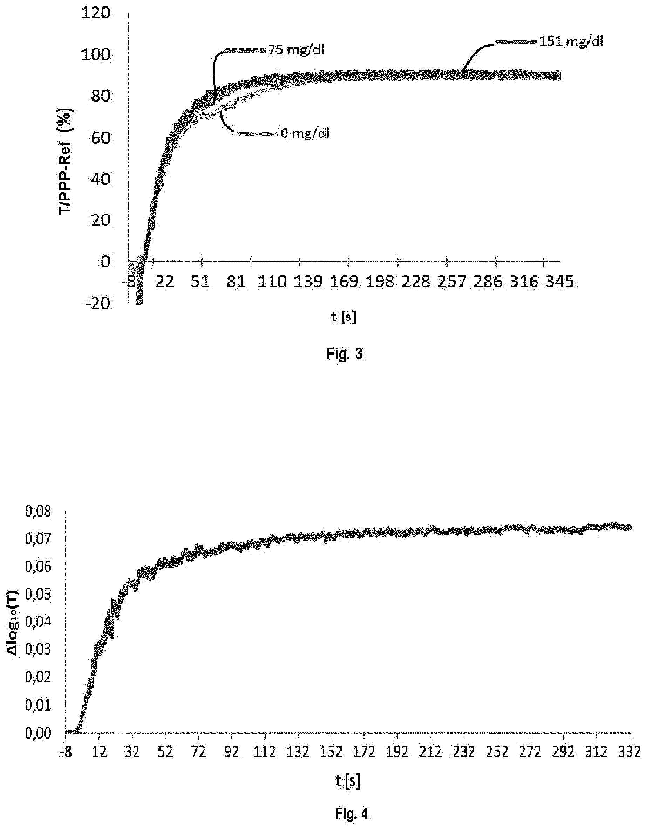

Der Absolutwert der Änderung des Transmissionsgrades hat für sich genommen keine Aussagekraft, da er beispielsweise von der Konzentration der in der Probe enthaltenen Thrombozyten sowie von der Konzentration und Art von anderen enthaltenen Inhaltsstoffen abhängt. Aus diesem Grund ist es im Stand der Technik erforderlich, jeweils einen PPP-Referenzwert durch eine Lichttransmissionsmessung am PPP derselben Blutprobe zu ermitteln und die am PRP erhaltenen Messwerte zu diesem gemessenen PPP-Referenzwert ins Verhältnis zu setzen. Solche PPP-Referenzwerte wurden bei den oben gezeigten Referenzblutproben auf aus dem Stand der Technik bekannte Weise ermittelt. Figur 3 zeigt das entsprechende Verhältnis der Transmissionsgrade der Referenzblutproben 1 bis 3 zum jeweiligen PPP-Referenzwert im Zeitverlauf. Es ist erkennbar, dass sich das Verhältnis im Zeitverlauf bei allen drei Proben einem Wert von etwa 85 annähert. Aus den in Figur 3 gezeigten Messdaten können Rückschlüsse auf die Funktionsfähigkeit der Thrombozyten gezogen werden.

Das erfindungsgemäße Verfahren zur Erstellung einer Datenbank wird nachfolgend anhand der in den Figuren 1 bis 3 gezeigten Messdaten beispielhaft erläutert. Zusätzlich zu den in den Figuren 1 bis 3 gezeigten Transmissionsmessungen wurde kurz vor Zugabe des Aktivators (bei t=-3s) sowie kurz nach Zugabe des Aktivators (durch Bildung eines Mittelwerts im Zeitraum zwischen t=2s und 6s) am PRP derselben Referenzblutproben jeweils eine Lichttransmissions-messung mit der Wellenlänge $\lambda2 = 405$ nm durchgeführt. Folgende Messergebnisse wurden beispielsweise für die Referenzblutprobe 1 ermittelt:

- der Transmissionsgrad bei der Wellenlänge $\lambda1$ vor Zugabe des Aktivators zum Zeitpunkt t=-3s: **$xd1 = 0,82676788$;**

- der Transmissionsgrad bei der Wellenlänge $\lambda2$ vor Zugabe des Aktivators zum Zeitpunkt t=-3s: **$xd2 = 0,532220558$;**

- der Transmissionsgrad bei der Wellenlänge $\lambda1$ nach Zugabe des Aktivators, erhalten durch Mittelwertbildung im Zeitraum zwischen t=2s und t=6s: **$xd3 = 0,840990463$;**

- der Transmissionsgrad bei der Wellenlänge $\lambda2$ nach Zugabe des Aktivators, erhalten durch Mittelwertbildung im Zeitraum zwischen t=2s und t=6s: **$xd4 = 0,551279411$,** sowie

- der PPP-Referenzwert gemessen am PPP der Referenzblutprobe: PPP-Ref = 1,0228.

Die Werte **$xd1$, $xd2$, $xd3$, $xd4$** und der PPP-Referenzwert wurden einer Datenbank hinzugefügt. Entsprechende Werte wurden auch für die Referenzblutproben 2 und 3 ermittelt und ebenfalls der Datenbank hinzugefügt. Die Datenbank ist in der nachfolgenden Tabelle 1 illustriert.

Tabelle 1

| Proben- Nr. | 1 | 2 | 3 |
|---|---|---|---|
| zugegebener Inhaltsstoff | - | Intralipid 75 mg/dl | Intralipid 151 mg/dl |
| **$xd1$** | 0,82676788 | 0,362703212 | 0,166929278 |
| **$xd2$** | 0,532220558 | 0,092530236 | 0,027191926 |
| **$xd3$** | 0,840990463 | 0,38363385 | 0,188179259 |
| **$xd4$** | 0,551279411 | 0,102101591 | 0,027871212 |
| PPP-Ref | 1, 0228 | 0,4433 | 0,2118 |
| virtREF | 1,0409 | 0,460 | 0,205 |

Entsprechende Messwerte wurden für weitere Referenzblutproben 4 bis 7 erfasst und ebenfalls der Datenbank hinzugefügt. Die Referenzblutproben 4 bis 7 unterschieden sich voneinander lediglich hinsichtlich der dem PRP jeweils manuell zugegebenen Inhaltsstoffe. Die Inhaltsstoffe sowie deren Konzentration sind zusammen mit den erfassten Messwerten in der nachfolgenden Tabelle 2 angegebenen.

Tabelle 2

| Proben-Nr. | 4 | 5 | 6 | 7 |
|---|---|---|---|---|
| Zugabe Inhaltsstoff | - | 15 mg/dl Bilirubin | 568 mg/dl Hämoglobin | 372 mg/dl Triglyceride |
| **$xd1$** | 0, 91048 | 0,881060 | 0,852417 | 0,557092 |

(fortgesetzt)

| Proben-Nr. | 4 | 5 | 6 | 7 |
|---|---|---|---|---|
| Zugabe Inhaltsstoff | - | 15 mg/dl Bilirubin | 568 mg/dl Hämoglobin | 372 mg/dl Triglyceride |
| **xd2** | 0, 56510 | 0,017670 | 0,129883 | 0,214720 |
| **xd3** | 0, 92432 | 0, 88447 | 0,862035 | 0,593829 |
| **xd4** | 0,58818 | 0,018583 | 0,151792 | 0,236192 |
| PPP-Ref | 1,0184 | 1,0160 | 0,9520 | 0, 6849 |
| virtREF | 1,01991 | 1, 01034 | 0,9831 | 0, 69194 |

Innerhalb der Datenbank sind Muster erkennbar, die auf dem Einfluss der zusätzlich hinzugefügten Inhaltsstoffe beruhen. Ein Vergleich der Transmissionswerte lässt beispielsweise erkennen, dass die Zugabe eines Inhaltsstoffs einen spezifischen Einfluss sowohl auf die ermittelten Transmissionswerte, als auch auf die gemessenen PPP-Referenzwerte haben kann. Beispielsweise zeigt ein Vergleich der Werte **xd1** und **xd2** der Proben 4 bis 6, dass eine durch die Zugabe von Bilirubin oder Hämoglobin ausgelöste Veränderung des Transmissionsgrades bei der Wellenlänge λ2 deutlich ausgeprägter ist als bei der Wellenlänge λ1.

Ein weiteres erkennbares Muster ist beispielsweise, dass bei der Probe 1 die Zugabe des Aktivators nur eine geringe Veränderung zwischen den Transmissionswerten **xd1** und **xd3** verursacht, während die Veränderung bei den Proben 2 und 3 (also mit Zunahme der Intralipidkonzentration) deutlich zunimmt (siehe Figur 2). Gleichzeitig führt die Zugabe der Inhaltsstoffe zu einer mehr oder weniger stark ausgeprägten Veränderung des PPP-Referenzwerts.

Nachdem die oben beispielhaft erläuterten Messungen an einer Vielzahl von Referenzblutproben durchgeführt und in die Datenbank aufgenommen wurden, führt die vorstehend beschriebene Art von Mustern zu statistisch signifikanten Zusammenhängen, die es ermöglichen, einen mathematischen Zusammenhang zur Ermittlung einer virtuellen Referenzgröße aufzustellen.

Ein beispielhafter mathematischer Zusammenhang wird nachfolgend erläutert:
Es werden die Größen:

$$V1 = xd1/xd2,$$

$$V2 = xd3/xd4,$$

$$V3 = V2/V1,$$

$$Ve = (V1 + V2)/e^{V3}$$

und

$$X^{xdPIP} = xd4 + xd3 - xd2 - xd1$$

ermittelt. Darüber hinaus wird die Größe

$$X^{vPIP} = (1 - xd3) \times (-\log_{10}(xd3)) \times (V1 + Ve)/e^{Ve}$$

ermittelt.. Anhand der vorstehend bezeichneten Größen kann eine vorläufige virtuelle Referenzgröße **1virtPPP** wie folgt ermittelt werden:

$$1virtPPP = 1 + X^{xdPIP} - X^{vPIP}.$$

Zudem wird der Faktor

$$F1 = xd3/1virtPPP$$

berechnet. Es hat sich gezeigt, dass für solche PRP-Proben, bei denen der Wert des Faktors **F1** kleiner ist als ein Schwellwert ist (im vorliegenden Beispiel **F1 < 1,19**), die vorläufige virtuelle Referenzgröße **1virtPPP** einen guten Schätzwert für den PPP-Referenzwert darstellt.

Sofern der Faktor **F1** größer ist als der Schwellwert (im vorliegenden Beispiel also **F1> 1,19**), so wird ein korrigierter virtuelle Referenzwert **2virtPPP** wie folgt ermittelt:

$$2virtPPP = 1{,}26 \times F1^{-1{,}134}.$$

Für solche PRP-Proben, bei denen der Faktor **F1 > 1,19** ist, stellt der korrigierte virtuelle Referenzwert **2virtPPP** einen guten Schätzwert für den PPP-Referenzwert dar. Aus der Datenbank wurde vorliegend somit der nachfolgend wiedergegebene mathematische Zusammenhang zur Bestimmung des virtuellen Referenzwerts **virtPPP** ermittelt:

$$virtPPP = \begin{cases} 1virtPPP = 1 + X^{xdPIP} - X^{vPIP}, falls\ F1 < 1{,}19 \\ 2virtPPP = 1{,}26 \times F1^{-1{,}134}, falls\ F1 > 1{,}19 \end{cases}$$

Die auf diese Weise für die Referenzproben 1 bis 6 ermittelten virtuellen Referenzwerte **virtPPP** sind in den vorstehenden Tabellen 1 und 2 angegeben. In den Tabellen ist zu sehen, dass eine gute Übereinstimmung mit den gemessenen PPP-Referenzwerten (PPP-Ref) besteht.

Figur 4 zeigt eine LTA-Messung am PRP einer zu untersuchenden Blutprobe, die unter denselben Messbedingungen wie die in den Figuren 1 und 2 gezeigten Messungen in einem Zeitraum zwischen t=-8s und t=6 min durchgeführt wurde, wobei die Messkurve zum Zeitpunkt t=-8s zum Nullpunkt verschoben ist. Zum Zeitpunkt t=0s wurde dem PRP im Verhältnis 9:1 der Aktivator ADP beigegeben. Die nachfolgenden Transmissionswerte **xd1, xd2, xd3, xd4** wurden für die Wellenlängen $\lambda1$=625nm, $\lambda2$=405nm auf die oben bereits beschriebene Weise jeweils vor und nach Zugabe des Aktivators bestimmt:

**xd1 = 0,698934, xd2 = 0,257505, xd3 = 0,726952, xd4 = 0,280035.**

Mittels des oben beschriebenen mathematischen Zusammenhangs wurden die oben genannten Zwischenwerte folgt berechnet:

| V1 | V2 | V3 | $X^{x}$dPIP | $V^{e}$ | $X^{v}$PIP |
|---|---|---|---|---|---|
| 2,714 | 2,596 | 0,956 | 0,05055 | 2,04055443 | 0,0264134 |

Der vorläufige virtuelle Referenzwert betrug:

**1virtPPP = 1,0259**

Zudem wurde der Faktor bestimmt.

**F1= 1,411** .

Da der Faktor den Wert von 1,19 übersteigt, ergab sich als virtueller Referenzwert:

$$virtPPP = 2virtPPP = 1{,}26 \times F1^{-1{,}134} = 0{,}857514.$$

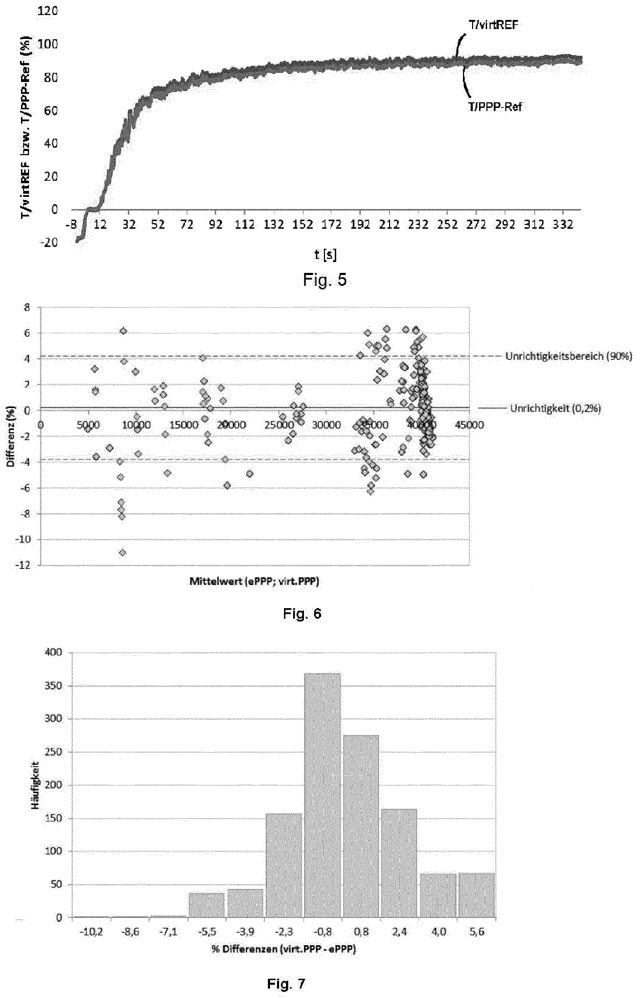

Zur Kontrolle wurde am PPP derselben zu untersuchenden Blutprobe auf aus dem Stand der Technik bekannte Weise ein PPP-Referenzwert PPP-Ref=0,8869 bestimmt. Der virtuelle Referenzwert stellt somit einen guten Schätzwert für den PPP-Referenzwert dar. Figur 5 zeigt das Verhältnis des gemessenen Transmissionswertes zum virtuellen Referenzwert virtREF sowie zum gemessenen Referenzwert PPP-Ref im Zeitverlauf. Aufgrund der guten Übereinstimmung der Werte virtREF und PPP-Ref sind die beiden in Figur 5 gezeigten Messkurven nahezu identisch.

Auf die oben beschriebene Weise wurde unter Verwendung des anhand der erfindungsgemäßen Datenbank ermittelten mathematischen Zusammenhangs für eine Mehrzahl von zu untersuchenden Blutproben jeweils ein virtueller Referenzwert (in der Figur als virt.PPP bezeichnet) ermittelt und zudem auf aus dem Stand der Technik bekannte Weise ein PPP-Referenzwert gemessen (in der Figur als ePPP bezeichnet), um den virtuellen Referenzwert jeweils mit dem gemessenen PPP-Referenzwert zu vergleichen. Dazu wurde der Mittelwert aus dem gemessenen PPP-Referenzwert und dem virtuellen Referenzwert ermittelt und die prozentuale Differenz zwischen den Größen bestimmt. Figur 6 zeigt diese Differenz als Funktion des Mittelwerts, wobei in der gezeigten Skala ein Mittelwert von 40000 in etwa einem PPP-Referenzwert von 1 entspricht. Es zeigt sich, dass mit Hilfe des erfindungsgemäßen Verfahrens für einen Großteil der untersuchten Blutproben ein virtueller Referenzwert berechnet werden konnte, der einen sehr guten Schätzwert für den tatsächlich gemessenen PPP-Referenzwert darstellt. Wie in Figur 7 illustriert ist, liegt die Differenz in 90% der Fälle innerhalb eines geringen Fehlerintervalls von etwa +/- 4%. Die Eignung des erfindungsgemäßen Verfahrens zur Bestimmung eines guten Schätzwertes für den PPP-Referenzwert wurde damit bestätigt.

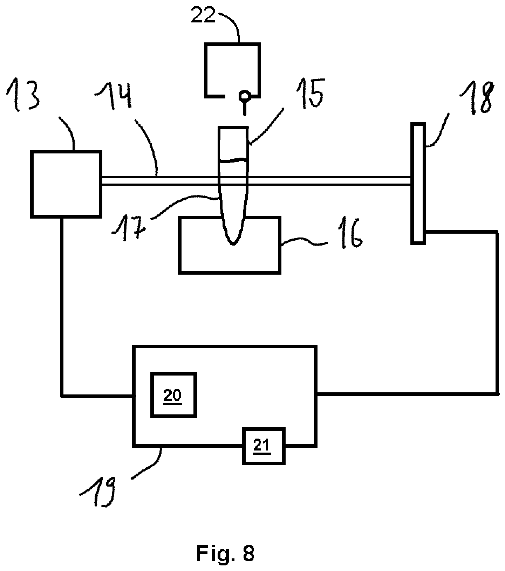

Figur 8 zeigt eine erfindungsgemäße Vorrichtung zur Ermittlung eines virtuellen Referenzwerts für die Durchführung einer LTA-Messung. Die Vorrichtung umfasst ein Beleuchtungsmodul 13, welches zur Aussendung von Lichtstrahlen 14 einer ersten Lichtwellenlänge von 405 nm und einer zweiten Lichtwellenlänge von 625 nm sowie mit jeweils vorgegebener Intensität ausgebildet ist. Die Vorrichtung umfasst weiterhin einen Probenhalter 16, in den eine transparente Küvette 15 manuell oder auch automatisch einsetzbar ist. In der Küvette 15 befindet sich PRP 17 einer zu untersuchenden Blutprobe. Der Probenhalter ist derart angeordnet, dass die Lichtstrahlen 14 auf einen Teilbereich der Küvette 15 Auftreffen und durch das darin angeordnete PRP 17 hindurchtreten. Die durch das PRP 17 hindurchgetretenen Lichtstrahlen 14 treffen anschließend auf einen Sensor 18, der die Intensität der Lichtstrahlen erfasst und an eine Steuermodul 19 weiterleitet. Die Vorrichtung weist weiterhin einen Applikator 22, der zur Zugabe einer vorgegebenen Menge eines Aktivators zum PRP 17 ausgebildet ist. Das Beleuchtungsmodul 13 und der Applikator 22 werden von der Steuermodul 19 zur Durchführung des erfindungsgemäßen Verfahrens gesteuert, wobei gleichzeitig die jeweiligen gemessenen Transmissionswerte xd1, xd2, xd3 und xd4 vom Sensor erfasst und im Steuermodul abgelegt werden. Das Steuermodul 19 umfasst weiterhin ein Rechenmodul 20, in dem der erfindungsgemäße mathematische Zusammenhang abgelegt ist. Unter Verwendung des mathematischen Zusammenhangs und der gemessenen Transmissionswerte ermittelt das Rechenmodul 20 einen virtuellen Referenzwert für die LTA Messung.

Alternativ oder zusätzlich kann auch eine Netzwerkschnittstelle 21 vorgesehen sein, welche die vom Sensor erfassten Messwerte xd1 bis xd4 über eine Datenverbindung zu einem externen Rechenmodul sendet, wobei die Ermittlung eines virtuellen Referenzwerts in diesem Fall vom externen Rechenmodul übernommen wird.

## Patentansprüche

1.  Verfahren zur Erstellung einer Datenbank zur Ermittlung eines virtuellen Referenzwerts für einen PPP-Referenzwert einer Lichttransmissionsaggregometrie-Messung, umfassend die nachfolgenden Schritte:

    a. Bereitstellen von plättchenreichem Plasma "PRP" ei - ner Referenzblutprobe;
    b. Durchführen einer Lichttransmissionsmessung mit einer ersten Lichtwellenlänge und einer zweiten von der ersten verschiedenen Lichtwellenlänge am PRP der Referenzblutprobe, wobei die erste Wellenlänge in einem Bereich zwischen 300 nm und 500 nm und die zweite Wellenlänge in einem Bereich zwischen 500 nm und 800 nm liegt, wobei sich die erste Lichtwellenlänge von der zweiten Lichtwellenlänge um mindestens 50 nm unterscheidet;
    c. Bereitstellen von plättchenarmem Plasma "PPP" der Referenzblutprobe;
    d. Durchführen einer Lichttransmissionsmessung am PPP zur Ermittlung eines PPP-Referenzwerts;
    e. Zuordnen des Messergebnisses des Schrittes d zu den Messergebnissen des Schrittes b in einer Datenbank;
    f. Wiederholen der Schritte a bis e für eine Mehrzahl von Referenzblutproben, die sich hinsichtlich der Art und/oder Konzentration von zumindest einem ihrer Inhaltsstoffe jeweils paarweise voneinander unterscheiden.

2.  Verfahren gemäß Anspruch 1, bei dem die erste und/oder zweite Wellenlänge zumindest eines der nachfolgenden Merkmale erfüllen:

    - die erste Wellenlänge unterscheidet sich von der zweiten Wellenlänge um mindestens 100 nm, vorzugsweise um mindestens 200 nm,
    - die erste Wellenlänge liegt in einem Bereich zwischen 345 nm und 465 nm, weiter vorzugsweise zwischen 385 nm und 425 nm, und
    - die zweite Wellenlänge liegt in einem Bereich zwischen 550 nm und 700 nm, weiter vorzugsweise wischen 600 nm und 640 nm.

3.  Verfahren gemäß Anspruch 1 oder 2, umfassend die weiteren Schritte:

    g. Zugeben einer vorgegebenen Menge eines Aktivators zum PRP der Referenzblutprobe nach dem Durchführen der Messung gemäß Schritt b;
    h. Wiederholen der Messung gemäß Schritt b nach dem Zugeben des Aktivators und vor dem Einsetzen einer durch den Aktivator ausgelösten Aggregation;
    i. Zuordnen des Messergebnisses des Schrittes h zu dem Messergebnis des Schrittes d in der Datenbank;
    j. Wiederholen der Schritte g bis i für die Mehrzahl von Referenzblutproben.

4.  Verfahren gemäß Anspruch 3, bei dem die Lichttransmissionsmessung gemäß Schritt h in einem Zeitraum zwischen 0 und 10s, vorzugsweise zwischen 0 und 5s nach der Zugabe des Aktivators durchgeführt wird.

5. Verfahren gemäß Anspruch 4, bei dem während der Lichttransmissionsmessung gemäß Schritt h ein zeitlicher Mittelwert der Lichttransmission über einen Zeitraum zwischen 1s und 6s, vorzugsweise zwischen 3s und 5s gebildet wird.

6. Verfahren gemäß einem der Ansprüche 1 bis 5, bei dem die Lichttransmissionsmessungen des Schrittes b mit zumindest drei voneinander verschiedenen Lichtwellenlängen durchgeführt werden.

7. Verfahren gemäß einem der Ansprüche 1 bis 6, bei dem die Mehrzahl von Referenzblutproben eine erste Referenzblutprobe und ein zweite Referenzblutprobe umfasst, wobei die erste Referenzblutprobe zumindest einen ersten Inhaltsstoff aufweist, der in der zweiten Referenzblutprobe nicht oder in einer Konzentration enthalten ist, die um einen Faktor von mehr als 1.5, vorzugsweise um einen Faktor von mehr als 3, weiter vorzugsweise um einen Faktor von mehr als 5 kleiner ist als die Konzentration des ersten Inhaltsstoffs in der zweiten Referenzblutprobe, wobei der erste Inhaltsstoff vorzugsweise ausgewählt ist aus der Gruppe bestehend aus Hämoglobin, Ceruloplasmin, Lipoprotein, Triglyceride, Bilirubin.

8. Verfahren gemäß Anspruch 7, bei dem der erste Inhaltsstoff der ersten Referenzblutprobe manuell hinzugefügt wird.

9. Verfahren zur Ermittlung eines virtuellen Referenzwerts für einen PPP-Referenzwert einer Lichttransmissionsaggregometrie-Messung am PRP einer zu untersuchenden Blutprobe unter Verwendung einer Datenbank gemäß einem der Ansprüche 1 bis 8, umfassend die nachfolgenden Schritte:

   a. Bereitstellen von PRP der zu untersuchenden Blutprobe;
   b. Durchführen einer Lichttransmissionsmessung mit einer ersten Lichtwellenlänge und einer zweiten von der ersten verschiedenen Lichtwellenlänge am PRP der zu untersuchenden Blutprobe;
   c. Verwenden der durch den Schritt b erhaltenen Messergebnisse sowie der Datenbank zur Ermittlung des virtuellen Referenzwerts der zu untersuchenden Blutprobe.

10. Verfahren gemäß Anspruch 9, weiterhin umfassend die Schritte:

   d. Zugeben einer vorgegebenen Menge eines Aktivators zum PRP der zu untersuchenden Blutprobe nach dem Durchführen der Messung gemäß Schritt b;
   e. Wiederholen der Messung gemäß Schritt b nach dem Zugeben des Aktivators zum PRP der zu untersuchenden Blutprobe und vor dem Einsetzen einer durch den Aktivator ausgelösten Aggregation;
   f. Einbeziehen der durch den Schritt e erhaltenen Messergebnisse in die Ermittlung des Schrittes c.

11. Verfahren gemäß einem der Ansprüche 9 oder 10, bei dem ein auf der Datenbank beruhender mathematischer Zusammenhang aufgestellt wird, der als Eingangsgrößen die an der zu untersuchenden Blutprobe gewonnenen Messergebnisse und als Ausgangsgröße den virtuellen Referenzwert der zu untersuchenden Blutprobe aufweist.

12. Verfahren zur Durchführung einer Lichttransmissionsaggregometrie-Messung an einer zu untersuchenden Blutprobe, umfassend die nachfolgenden Schritte:

   a. Durchführen des Verfahrens eines der Ansprüche 9 bis 11 zur Ermittlung des virtuellen Referenzwerts der zu untersuchenden Blutprobe,
   b. Durchführen einer Lichttransmissionsaggregometrie-messung unter Verwendung des virtuellen Referenzwerts.

13. Vorrichtung zur Ermittlung eines virtuellen Referenzwerts für die Durchführung einer LTA-Messung am PRP einer zu untersuchenden Blutprobe, umfassend ein Beleuchtungsmodul (13) zum wahlweisen Aussenden von Licht einer ersten sowie einer zweiten von der ersten verschiedenen Lichtwellenlänge, eine Probenaufnahme (16) zum Einsetzen einer PRP-Probe (17) derart, dass das Licht des Beleuchtungsmoduls (13) durch das PRP (17) hindurchtritt, einen Lichtsensor (18), der zur Erfassung des durch das PRP (17) hindurchgetretenen Lichts ausgebildet ist, sowie ein Steuermodul (19), welches dazu ausgebildet ist, die Vorrichtung derart anzusteuern, dass die folgenden Schritte ausgeführt werden:

   a. Durchführen einer Lichttransmissionsmessung mit einer ersten Lichtwellenlänge und einer zweiten von der ersten verschiedenen Lichtwellenlänge am PRP der zu untersuchenden Blutprobe, wobei die erste Wellenlänge in einem Bereich zwischen 300 nm und 500 nm und die zweite Wellenlänge in einem Bereich zwischen 500 nm und 800 nm liegt, wobei sich die erste Lichtwellenlänge von der zweiten Lichtwellenlänge um mindestens 50 nm unterscheidet;

b. Verwenden der durch den Schritt a erhaltenen Messergebnisse sowie einer Datenbank gemäß einem der Ansprüche 1 bis 8 zur Ermittlung eines virtuellen Referenzwerts der zu untersuchenden Blutprobe.

14. Vorrichtung gemäß Anspruch 13, welche einen Applikator (22) zum automatischen Zugeben einer vorgegebenen Menge eines Aktivators zum PRP (17) aufweist, wobei das Steuermodul (19) dazu ausgebildet ist, die Vorrichtung derart anzusteuern, dass die folgenden Schritte ausgeführt werden:

c. Zugeben einer vorgegebenen Menge eines Aktivators zum PRP der zu untersuchenden Blutprobe nach dem Durchführen der Messung gemäß Schritt a;

d. Wiederholen der Messung gemäß Schritt a nach dem Zugeben des Aktivators zum PRP der zu untersuchenden Blutprobe und vor dem Einsetzen einer durch den Aktivator ausgelösten Aggregation;

e. Einbeziehen der durch den Schritt d erhaltenen Messergebnisse in die Ermittlung des Schrittes b.

**Claims**

1. Method for creating a database for determining a virtual reference value for a PPP reference value of a light transmission aggregometry measurement, comprising the following steps:

a. providing platelet-rich plasma "PRP" of a reference blood sample;

b. performing a light transmission measurement with a first light wavelength and a second light wavelength, different from the first, on the PRP of the reference blood sample, wherein the first wavelength is in a range between 300 nm and 500 nm and the second wavelength is in a range between 500 nm and 800 nm, wherein the first light wavelength differs from the second light wavelength by at least 50 nm;

c. providing platelet-poor plasma "PPP" of the reference blood sample;

d. performing a light transmission measurement on the PPP in order to determine a PPP reference value;

e. assigning the measurement result of step d to the measurement results of step b in a database;

f. repeating steps a to e for a plurality of reference blood samples that each differ from one another in pairs in terms of the type and/or concentration of at least one of their substances.

2. Method according to Claim 1, in which the first and/or second wavelength satisfy at least one of the following features:

- the first wavelength differs from the second wavelength by at least 100 nm, preferably by at least 200 nm,
- the first wavelength is in a range between 345 nm and 465 nm, more preferably between 385 nm and 425 nm, and
- the second wavelength is in a range between 550 nm and 700 nm, more preferably between 600 nm and 640 **nm.**

3. Method according to Claim 1 or 2, comprising the following further steps:

g. adding a predefined amount of an activator to the PRP of the reference blood sample after performing the measurement according to step b;

h. repeating the measurement according to step b after adding the activator and before introducing an aggregation triggered by the activator;

i. assigning the measurement result of step h to the measurement result of step d in the database;

j. repeating steps g to i for the plurality of reference blood samples.

4. Method according to Claim 3, in which the light transmission measurement according to step h is performed in a time period between 0 and 10 s, preferably between 0 and 5 s after the addition of the activator.

5. Method according to Claim 4, in which, during the light transmission measurement according to step h, a temporal average value of the light transmission is formed over a time period between 1 s and 6 s, preferably between 3 s and 5 s.

6. Method according to one of Claims 1 to 5, in which the light transmission measurements of step b are performed with at least three mutually different light wavelengths.

7. Method according to one of Claims 1 to 6, in which the plurality of reference blood samples comprises a first reference blood sample and a second reference blood sample, wherein the first reference blood sample contains at least one first substance that is not contained in the second reference blood sample or is contained in the second reference blood sample in a concentration that is less by a factor of more than 1.5, preferably by a factor of more than 3, more preferably by a factor of more than 5 than the concentration of the first substance in the second reference blood sample, wherein the first substance is preferably selected from the group consisting of hemoglobin, ceruloplasmin, lipoprotein, triglycerides, bilirubin.

8. Method according to Claim 7, in which the first substance of the first reference blood sample is added manually.

9. Method for determining a virtual reference value for a PPP reference value of a light transmission aggregometry measurement on the PRP of a blood sample to be examined using a database according to one of Claims 1 to 8, comprising the following steps:

a. providing PRP of the blood sample to be examined;
b. performing a light transmission measurement with a first light wavelength and a second light wavelength, different from the first, on the PRP of the blood sample to be examined;
c. using the measurement results obtained by step b and the database to determine the virtual reference value of the blood sample to be examined.

10. Method according to Claim 9, furthermore comprising the following steps:

d. adding a predefined amount of an activator to the PRP of the blood sample to be examined after performing the measurement according to step b;
e. repeating the measurement according to step b after adding the activator to the PRP of the blood sample to be examined and before introducing an aggregation triggered by the activator;
f. incorporating the measurement results obtained by step e into the determination of step c.

11. Method according to either of Claims 9 and 10, in which a mathematical relationship based on the database is established, this relationship having the measurement results obtained on the blood sample to be examined as input variables and the virtual reference value of the blood sample to be examined as output variable.

12. Method for performing a light transmission aggregometry measurement on a blood sample to be examined, comprising the following steps:

a. performing the method from one of Claims 9 to 11 for determining the virtual reference value of the blood sample to be examined,
b. performing a light transmission aggregometry measurement using the virtual reference value.

13. Device for determining a virtual reference value for performing an LTA measurement on the PRP of a blood sample to be examined, comprising an illumination module (13) for selectively emitting light of a first light wavelength and of a second light wavelength different from the first, a sample receptacle (16) for the introduction of a PRP sample (17) such that the light from the illumination module (13) passes through the PRP (17), a light sensor (18) that is designed to capture the light that has passed through the PRP (17), and a control module (19) that is designed to drive the device such that the following steps are carried out:

a. performing a light transmission measurement with a first light wavelength and a second light wavelength, different from the first, on the PRP of the blood sample to be examined, wherein the first wavelength is in a range between 300 nm and 500 nm and the second wavelength is in a range between 500 nm and 800 nm, wherein the first light wavelength differs from the second light wavelength by at least 50 nm;
b. using the measurement results obtained by step a and a database according to one of Claims 1 to 8 to determine a virtual reference value of the blood sample to be examined.

14. Device according to Claim 13, having an applicator (22) for automatically adding a predefined amount of an activator to the PRP (17), wherein the control module (19) is designed to drive the device such that the following steps are carried out:

c. adding a predefined amount of an activator to the PRP of the blood sample to be examined after performing the measurement according to step a;

d. repeating the measurement according to step a after adding the activator to the PRP of the blood sample to be examined and before introducing an aggregation triggered by the activator;

e. incorporating the measurement results obtained by step d into the determination of step b.

**Revendications**

1. Procédé de création d'une base de données pour la détermination d'une valeur de référence virtuelle pour une valeur de référence de PPP d'une mesure d'agrégométrie par transmission lumineuse, comprenant les étapes suivantes :

a. la fourniture de plasma riche en plaquettes « PRP » d'un échantillon sanguin de référence ;
b. la réalisation d'une mesure de transmission lumineuse sur le PRP de l'échantillon sanguin de référence à une première longueur d'onde lumineuse et à une seconde longueur d'onde lumineuse qui diffère de la première, la première longueur d'onde se situant dans une plage comprise entre 300 nm et 500 nm et la seconde longueur d'onde se situant dans une plage comprise entre 500 nm et 800 nm, la première longueur d'onde lumineuse différant de la seconde longueur d'onde lumineuse d'au moins 50 nm ;
c. la fourniture de plasma pauvre en plaquettes « PPP » de l'échantillon sanguin de référence ;
d. la réalisation d'une mesure de transmission lumineuse sur le PPP pour la détermination d'une valeur de référence de PPP ;
e. l'association du résultat de mesure de l'étape d aux résultats de mesure de l'étape b dans une base de données ;
f. la répétition des étapes a à e pour une pluralité d'échantillons sanguins de référence qui diffèrent les uns des autres par paires en ce qui concerne le type et/ou la concentration d'au moins l'un de leurs constituants.

2. Procédé selon la revendication 1, dans lequel la première et/ou la seconde longueur d'onde présentent au moins l'une des caractéristiques suivantes :

- la première longueur d'onde diffère de la seconde longueur d'onde d'au moins 100 nm, de préférence d'au moins 200 nm,
- la première longueur d'onde se situe dans une plage comprise entre 345 nm et 465 nm, plus préférentiellement entre 385 nm et 425 nm, et
- la seconde longueur d'onde se situe dans une plage comprise entre 550 nm et 700 nm, plus préférentiellement entre 600 nm et 640 nm.

3. Procédé selon la revendication 1 ou 2, comprenant les étapes supplémentaires suivantes :

g. l'ajout d'une quantité prédéfinie d'un activateur au PRP de l'échantillon sanguin de référence après la réalisation de la mesure selon l'étape b ;
h. la répétition de la mesure selon l'étape b après l'ajout de l'activateur et avant le début d'une agrégation déclenchée par l'activateur ;
i. l'association du résultat de mesure de l'étape h au résultat de mesure de l'étape d dans la base de données ;
j. la répétition des étapes g à i pour la pluralité d'échantillons sanguins de référence.

4. Procédé selon la revendication 3, dans lequel la mesure de transmission lumineuse selon l'étape h est réalisée dans un laps de temps compris entre 0 et 10 s, de préférence entre 0 et 5 s, après l'ajout de l'activateur.

5. Procédé selon la revendication 4, dans lequel, pendant la mesure de transmission lumineuse selon l'étape h, une moyenne temporelle de la transmission lumineuse est calculée sur un laps de temps compris entre 1 s et 6 s, de préférence entre 3 s et 5 s.

6. Procédé selon l'une quelconque des revendications 1 à 5, dans lequel les mesures de transmission lumineuse de l'étape b sont réalisées à au moins trois longueurs d'onde lumineuses différentes les unes des autres.

7. Procédé selon l'une quelconque des revendications 1 à 6, dans lequel la pluralité d'échantillons sanguins de référence comprend un premier échantillon sanguin de référence et un second échantillon sanguin de référence, le premier échantillon sanguin de référence comprenant au moins un premier constituant qui n'est pas contenu dans le second échantillon sanguin de référence ou qui l'est à une concentration qui est inférieure d'un facteur supérieur à 1,5, de préférence d'un facteur supérieur à 3, plus préférentiellement d'un facteur supérieur à 5, à la concentration du premier constituant dans le second échantillon sanguin de référence, le premier constituant étant de préférence sélectionné dans le groupe constitué par l'hémoglobine, la céruloplasmine, une lipoprotéine, un triglycéride, la bilirubine.

8. Procédé selon la revendication 7, dans lequel le premier constituant du premier échantillon sanguin de référence est ajouté manuellement.

9. Procédé de détermination d'une valeur de référence virtuelle pour une valeur de référence de PPP d'une mesure d'agrégométrie par transmission lumineuse sur le PRP d'un échantillon sanguin à examiner en utilisant une base de données selon l'une quelconque des revendications 1 à 8, comprenant les étapes suivantes :

   a. la fourniture de PRP de l'échantillon sanguin à examiner ;
   b. la réalisation d'une mesure de transmission lumineuse à une première longueur d'onde lumineuse et à une seconde longueur d'onde lumineuse qui diffère de la première, sur le PRP de l'échantillon sanguin à examiner ;
   c. l'utilisation des résultats de mesure obtenus par l'étape b ainsi que de la base de données pour la détermination de la valeur de référence virtuelle de l'échantillon sanguin à examiner.

10. Procédé selon la revendication 9, comprenant en outre les étapes suivantes :

   d. l'ajout d'une quantité prédéfinie d'un activateur au PRP de l'échantillon sanguin à examiner après la réalisation de la mesure selon l'étape b ;
   e. la répétition de la mesure selon l'étape b après l'ajout de l'activateur au PRP de l'échantillon sanguin à examiner et avant le début d'une agrégation déclenchée par l'activateur ;
   f. l'intégration des résultats de mesure obtenus par l'étape e dans la détermination de l'étape c.

11. Procédé selon l'une quelconque des revendications 9 ou 10, dans lequel on établit à partir de la base de données une relation mathématique qui présente comme grandeurs d'entrée les résultats de mesure obtenus sur l'échantillon sanguin à examiner et comme grandeur de sortie la valeur de référence virtuelle de l'échantillon sanguin à examiner.

12. Procédé de réalisation d'une mesure d'agrégométrie par transmission lumineuse sur un échantillon sanguin à examiner, comprenant les étapes suivantes :

   a. la mise en œuvre du procédé selon l'une quelconque des revendications 9 à 11 pour la détermination de la valeur de référence virtuelle de l'échantillon sanguin à examiner,
   b. la réalisation d'une mesure d'agrégométrie par transmission lumineuse en utilisant la valeur de référence virtuelle.

13. Dispositif de détermination d'une valeur de référence virtuelle pour la réalisation d'une mesure de LTA sur le PRP d'un échantillon sanguin à examiner, comprenant un module d'éclairage (13) pour l'émission sélective de lumière à une première longueur d'onde et à une seconde longueur d'onde différente de la première, un logement à échantillon (16) pour l'insertion d'un échantillon de PRP (17) de sorte que la lumière provenant du module d'éclairage (13) traverse le PRP (17), un capteur de lumière (18) qui est conçu pour détecter la lumière ayant traversé le PRP (17), ainsi qu'un module de commande (19) qui est conçu pour commander le dispositif de manière à exécuter les étapes suivantes :

   a. la réalisation d'une mesure de transmission lumineuse à une première longueur d'onde lumineuse et à une seconde longueur d'onde lumineuse qui diffère de la première sur le PRP de l'échantillon sanguin à examiner, la première longueur d'onde se situant dans une plage comprise entre 300 nm et 500 nm et la seconde longueur d'onde se situant dans une plage comprise entre 500 nm et 800 nm, la première longueur d'onde lumineuse différant de la seconde longueur d'onde lumineuse d'au moins 50 nm ;
   b. l'utilisation des résultats de mesure obtenus par l'étape a ainsi que d'une base de données selon l'une quelconque des revendications 1 à 8 pour la détermination d'une valeur de référence virtuelle de l'échantillon sanguin à examiner.

14. Dispositif selon la revendication 13, comprenant un applicateur (22) permettant d'ajouter automatiquement une quantité prédéfinie d'un activateur au PRP (17), dans lequel le module de commande (19) est conçu pour commander le dispositif de manière à exécuter les étapes suivantes :

c. l'ajout d'une quantité prédéfinie d'un activateur au PRP de l'échantillon sanguin à examiner après la réalisation de la mesure selon l'étape a ;

d. la répétition de la mesure selon l'étape a après l'ajout de l'activateur au PRP de l'échantillon sanguin à examiner et avant le début d'une agrégation déclenchée par l'activateur ;

e. l'intégration des résultats de mesure obtenus par l'étape d dans la détermination de l'étape b.

Fig. 1

Fig. 2

Fig. 3

Fig. 4

Fig. 5

Fig. 6

Fig. 7

**Fig. 8**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2017248576 A1


**In der Beschreibung aufgeführte Nicht-Patentliteratur**

- Evaluation of the Newly Developed Adenosine Diphosphate induced Platelet Aggregation Level System. **SAKAYORI TASKUKU et al.** Aggregometer on Automated Coagulation Analyzer. CLINICAL LABORATORY, 01 December 2019
- **LING LI-QIN et al.** Evaluation of an automated light transmission aggregometry. *PLATELETS (LONDON)*, 02 February 2017, 712-719
- **LE BLANC JESSICA et al.** Advances in Platelet Function Tenting-Light Transmission Aggregometry and Beyond. *JOURNAL OF CLINICAL MEDICINE*, 13 August 2020, vol. 9 (8), 2636
- Mukaide Kae: ''Overview of the Automated Coagulation Analyzer CS5100. *Sysmex Journal International*, 2013
- Leitlinie-Thrombozytopathien, Version 2.1. *AWMF*, February 2018 (086-003)